# EUROPEAN PATENT APPLICATION

(11) **EP 1 878 450 A1**
(43) Date of publication of application: **16.01.2008**
(21) Application number: 06116882.9
(22) Date of filing: 10.07.2006
(51) Int. Cl.: A61L 27/44, A61L 27/46, A61L 27/56

(54) **Cellular gradient polymer composites**

(71) Applicant: ECOLE POLYTECHNIQUE FEDERALE DE LAUSANNE (EPFL), 1015 Lausanne (CH)
(72) Inventor: BOURBAN, Pierre-Etienne, 1260, Nyon (CH); BUHLER, Manuel, 9200, Gossau (CH); MANSON, Jan-Anders Edvin, 1071, Chexbres (CH); MATHIEU, Laurence Marcelle Monique, 1024, Ecublens (CH)
(74) Representative: Grosfillier, Philippe

(57) **Abstract**

The invention relates to a polymer composite product obtained by a process comprising the following steps :
- preparation of a composite preform by precise placement of dry- or preimpregnated- fillers and/or fibres in a mould,
- incorporation of the said preform and of a polymeric material in a foaming mould,
- foaming of the prepared composite

said polymer composite showing a gradient of filler and/or fibre content and a gradient of porosity.

The polymer composite according to the invention may advantageously be used in tissue engineering, bone replacement, consumer goods, transportation or in any other suitable field.

The invention also includes a process for manufacturing said polymer composite.

## Description

### FIELD OF THE INVENTION

This invention relates to cellular composite products based on polymers incorporating fillers and/or fibres and the method to process them. Such composite structures may advantageously be used in tissue engineering, bone replacement, consumer goods, transportation or in any other suitable field.

### BACKGROUND

Porous materials are numerous in nature (bone, wood, sponge....) as well as in synthetic materials (foams, honeycomb for sandwich structures,...). Bone structure for instance, has been optimised by nature during millions of years, offering performance in terms of lightness, stiffness, strength, shapes, porosity, healing performance etc. As bone is basically a composite of natural ceramic and polymer materials with different distribution of porosity and mechanical properties, it is of interest to mimic this material and structure.
Any synthetic composite offering the properties of natural bone or based on a similar microstructure will open new opportunities for the next generation of polymer composites such as for applications in the biomedical field where they can be used as optimised implant for example. Similar structures will as well offer novel opportunities in applications where lightness and strength are required.

### Porous polymers

Polymer foams are widely used in various applications such as insulation panels, furniture, damping components, composite sandwich structures... Mechanical properties of these cellular solids mainly depend on the morphology of the pores, the properties of the raw material and the density of the foam (Gibson and Ashby 1988). Several foaming processes have been developed according to the different polymer types (Klempner and Frisch 1991). Current research aims at a better understanding of the foaming mechanisms in order to better control distributions and gradients of pore sizes as well as their morphology development.

### Composite materials

Polymer based composites have been developed for their design freedom, offering tailored mechanical properties via the combination of polymers with different fillers or fibres (Manson 2000). Composition, orientation and architecture of the reinforcement can be selected to provide desired final performance. Several processing methods have been developed with always the same objective, obtaining a composite with a minimum of porosity in order to ensure optimum performance. Indeed uncontrolled morphology of pores induces stress concentrations, reductions of stiffness and early failure.

### Gradient materials

The main feature of gradient material is that its properties change gradually with position. The property gradient in the material is caused by a position dependent chemical composition, microstructure or atomic order. An early example of a gradient material manufactured by mankind is polyurethane skin foams, which are fully dense at the surface and porous in the interior. They provide high impact strength at low weight and are thus used for instrument panels or head rests in cars (Piechota and Röhr 1975). Another criterion can be the geometry of the gradation (throughout the bulk or only a coating) or the gradation's function as it is the case for functionally graded materials (FGMs). For processing FGMs numerous techniques are available. For metallic and ceramic materials, traditional powder processing can be used if an additional gradation step is introduced before consolidation. Wet processing takes use of aqueous suspensions instead of powders.
In polymer based composites, the sheet-lamination (stacking of 2D-fabrics) is used to obtain products with a step-like gradient through the thickness. Sometimes the impregnation distances in such composites are quite long, what motivated for example the development of preimpregnated preforms and of commingled (hybrid) yarns (Bourban, Bernet et al. 2001). Furthermore in sheet-lamination one might be confronted with delamination between laminae, stress concentrations and distributions, occurrence of internal stresses (Sunderland, Yu et al. 2001) or deconsolidation (Wolfrath, Michaud et al. 2005) due to the elastic energy stored in the fibre reinforcement network of the composite. The deconsolidation phenomena can be used for fabrication of porosity gradients in thermoplastic materials. Hereby the attainable porosity gradients are directly linked to the porosity increase during deconsolidation. This technique allows only very specific and limited design of polymer-based gradient composites. Indeed to precisely mimic bone structure for example larger porosity volumes, control of pore size and distribution, precise control of fillers and fibres positions are required. Combination of porosity and reinforcement gradients is still to be developed.
Indeed, bone is a very good example of a tailored porous composite structure. It has a complex hierarchical organization at the micro- and nano-level, and is made of hydroxyapatite crystals, collagen molecules and water as elementary components. Furthermore it shows a continuous change in architecture and collagen concentration when passing from cortical to trabecular bone tissue. There is an interphase in which the porosity passes from closed to open porosity. In this meaning bones are considered as functionally graded materials. Bones are viscoelastic and anisotropic. The current invention proposes composite systems and processing methods to mimic these bone properties.

### Polymers and composites in biomedical applications

Favourable materials such as stainless steel, titanium and its alloys, calcium phosphates and alumina ceramics, polyethylene (PE) and polyetheretherketon (PEEK), and composites of these materials have been used in orthopaedic surgery. At present biodegradable polymers are also being studied, and have had some clinical success, with the aim of avoiding a second operation after successful bone healing.
Polymer biomaterials have gained a significant importance in health care and well-being in our modem society (Chu 2000). Fibres and textile fabrics have found some applications in this field. Sutures in wound closure represent the first and major use of fibres in medicine. Textiles can have either external uses like bandages, or internal, like vascular grafts where they bring flexibility and porosity, or as reinforcement of polymer in hip prosthesis, where they can bring anisotropy and enable to better match bone properties. Textiles could also be used for scaffolds in tissue engineering (Hutmacher 2000). Competences in science and engineering are currently being gathered to develop functional tissue and organ replacements (Burg, Porter et al. 2000; Rose and Oreffo 2002). In scaffold applications, fibres have been used as non woven mesh, which provides high porosity and a large surface area for cell adhesion and proliferation. However this type of scaffold is highly deformable and has poor mechanical properties (Cima, Vacanti et al. 1991). Attempts have also been made to prepare scaffolds with 3D woven fabric, using Ultra High Molecular Weight Polyethylene (UHMWPE) fibres coated with Low Density Polyethylene (LDPE). This 3D textile has been tested in cartilage applications and has shown compatibility with surrounding tissues (Shikinami and Kawarada 1998). Wintermantel (Wintermantel, Mayer et al. 1996) also suggests that superstructures with knitted fibres could be used in tissue engineering: they are porous, deformable to obtain complex shapes, and stiffness can be increased by impregnation with a polymer. Research in tissue engineering is also conducted to provide synthetic tissue graft (Atala and Mooney 1997). Scaffolds for tissue engineering need to be highly porous to have a high specific surface area for cell adhesion and a high void volume to allow vascularisation, nutrient diffusion and cell proliferation and differentiation. The first idea for such a porous structure is open-cell foams. Four main techniques of foaming have been developed mainly for bioresorbable polymers, with their own advantages and drawbacks: solvent casting and leaching, gas foaming, emulsion freeze-drying and thermally induced phase separation. They can be adapted to thermoplastics, and more specifically to bioresorbable and biocompatible polymers. It is known that non medical grades of PE or PET foams, with closed cells, are used in packaging, sports, construction, and automotive. Biocompatible grades of these polymers and bioresorbable polymers will certainly be more extensively considered in future for biomedical applications when tailored open-cell structures can be produced.

It is to note that mechanical properties of polymer foams always appear to be low, in particular if intended use is hard tissue engineering. It could thus be interesting to prepare and evaluate three-dimensional open-cell foams, i.e. porous polymer, reinforced with fillers, fibres and/or textile fabrics. Furthermore, these reinforcements can bring anisotropy, gradient of properties and functions.

### Porous polymer composites for bone tissue engineering

Despite bone grafts is a routine procedure in orthopaedic surgery, no satisfactory bone substitutes are currently available. The use of synthetic bone substitutes is increasing; however, in a review of clinically available bone substitutes in France, no polymer scaffold was available for orthopaedic applications (Mainard, Gouin et al. 2001). Most of the synthetic bone grafts used are made of ceramic such as calcium phosphates, which do not allow load-bearing application due to their low toughness and brittle mechanical behaviour. There is actually a need for new synthetic bone scaffolds which can be used in different surgical situations, especially for load-bearing applications. Scaffolds based on porous polymer composites represent then an attractive solution.

The first step in bone and cartilage tissue engineering is to develop a scaffold with controlled **high porosity** for maximal loading with cells and tissue ingrowth. It should be able to support cell proliferation, differentiation, and function. For bone, the minimum pore diameter required for ingrowth of cells into the interior of the matrices is 100 µm (Laurencin, Attawia et al. 1996). Indeed, it has been suggested that the ideal pore-size range of 200-400 µm is preferred by osteoblasts because it provides the optimum compression and tension on the osteoblast's mechanoreceptor (Boyan, Hummert et al. 1996). One of the key aspects of size porosity is to avoid occlusion of pores by cells. **Interconnected porosity** is also important for bone ingrowth (Baksh and Davies 2000). However, diffusion does not seem related to pore sizes as for osteocytes, canaliculi of the micrometer sizes allowed nutrients to reach these cells. As bone formation needs vascular support, the scaffold should also allow a rapid development of vascularization.
From a **biomechanics** point of view, properties of the scaffold should be close to the ones of bone if load-bearing applications are planned. In the slow regeneration of skeletal tissues, the mechanical stress should be engineered to be low during early phases, although it should increase during the late differentiation phase to properly align and mature the mechanical functioning of the repair tissue. A high initial stiffness will allow primary union followed by gradual resorption and reduction in stiffness corresponding to simultaneous bone healing to take-up load-bearing functions.
Furthermore, the material should be easily processed into complex shaped components (USpatent0054372A1) even when bioresorbable constituents are used.

So far, in thermoplastic polymer foams mainly micro-sized, and more recently, nano-sized, fillers were used to reinforce foams and/or tailor foam morphology and properties. They were produced through various routes, such as reactive foaming of polyurethanes, solvent-phase processing, and gas foaming. The latter technique was in particular applied to a bioresorbable calcium phosphate - poly(L-lactic acid) (PLA) system, showing improved compression resistance and improved biocompatibility with bone cells (Mathieu, Montjovent et al. 2005; Mathieu, T et al. 2005; Mathieu, Mueller et al. 2006). The observed increase in mechanical properties is related to an increase in foam density.
Reactive foaming and solvent-based processes cited previously were used to obtain fibre-reinforced foams. However, particles and short fibres do not reinforce the polymeric foams sufficiently. Polyurethane (PUR) foams have already been reinforced with glass fibres, jute or woven flax (Bledzki A.K. 2001). However, to our knowledge no continuous fibres have been introduced into thermoplastic foams and an interconnected and micro-sized porosity obtained. Moreover no gradients in fibre volumes have yet been considered in the case of porous or cellular structures. The addition of continuous fibres, possibly combined with short fibres / particles in the matrix, allows further reinforcement even if the porosity is kept constant. By combining reinforcement volume and porosity gradients, structures can be tailored which are similar to the natural bone architecture, having an interface between trabecular and cortical bone, without having discontinuities in structure and concentration. The interest in the use of long, continuous reinforcing fibres in foams lies in the fact that the critical fibre length increases with the porosity and thus the mechanical foam properties (modulus, rupture resistance) can be increased.
Ideally the material should be easily processed into complex shaped components. A material exhibiting the viscoleastic and anisotropic properties of natural tissue will be preferred as it will integrate better into the body and transmit load in an optimised way reducing stress concentration or stiffness mismatches for instance.
Current ceramic implants have an intrinsic low toughness and can not easily be shaped or screwed; that is why they are not considered as ideal. There is also risk of inflammation induced by micro movements between implant and bone, because of modulus mismatch.

Biocompatible and bioresorbable polymers present instead ductile properties. For example, bioresorbable poly(L-lactic acid) (PLA) is a very good candidate for implants and bone replacing material. Fibres and/or fillers can be added to improve not only the mechanical behaviour but also other properties of the PLA such as dimensional or thermal stability, barrier properties, and biological functionalities. (USpatent5108755) discloses composites comprising Poly-ε-caprolactone matrix reinforced with certain biodegradable fibres for improved retention of yield strength and Young's modulus with time under degrading conditions. (USpatent4655777) is disclosing matrix reinforced with biodegradable fibres for increased strength of bone fixation plates in which no significant porosity , such as it would be the case for bone replacement implants, is present. The composite is prepared using conventional processing routes. As in the case of bulk composites, a similar extension of properties range can be obtained with porous composite structures.

In summary, mechanical performance of current scaffolds needs to be improved, and scaffolds should also be processable during surgery to correctly fit the bone defect.

Polymer based composites have the potential to mimic bone microstructure, to provide range of mechanical properties and offer design freedom.

There is therefore a need for a new technique which enables the fabrication of composites with a thermoplastic matrix and reinforcing elements such as fillers, short or long or continuous fibres and having at the same time reinforcement volume and porosity gradients.
The challenge is to combine fibre-volume and porosity gradients into composite structures which can be tailored to be similar to the natural bone architecture, having an interface between trabecular and cortical bone, without having discontinuities in structure and stress concentration.

### SUMMARY OF INVENTION

A porous and reinforced polymer composite product is proposed, preferably obtained through a solvent-free process comprising the precise placement of fibres and/or fillers followed by the gas foaming of the composite preform. The composite product has a controlled cellular structure based on polymers with fillers, short, long or continuous fibres and combination of them. Gradients of fibre volumes (when fibres are used) are controlled through the placement of filaments, yarns or commingled yams; fibres contents up to 65 vol % can be reached locally. Porosity can be varied from 0 up to 90 % in volume and porosity gradients are achieved by varying the gas foaming parameters. The process can combine different types of gradients in order to prepare polymer foams with tailored variations of filler/fibre volumes and of porosity in several directions of the product. Fibres improve the mechanical modulus and strength of the porous composite while providing desired anisotropy and functional properties.

The invention will be better understood below with a detailed description including examples illustrated by the following figures :
Fig 1: Schematic representation of cross-sections for novel cellular composite with gradients of fillers or short fibre volumes (type A), for cellular composite with 2D distribution of continuous fibres (type B) and with 3D distribution of continuous fibres (type C).
Fig 2: Winding set-up for precise placement of reinforcement and functional fibres or yarns for processing composite of type B.
Fig 3: Detailed representation of the mould for fibre placement and preconsolidation of type B composite
Fig 4: Holder to place and orient reinforcement and functional fibres or yarns for processing composites of type C.
Fig 5: Cross-sections of a type A composite showing gradient of porosity.
Fig 6: Processing window for preconsolidation and consolidation, both at 200°C.
Fig 7: Micrograph of a cross-section of a composite B with a fibre free centre, cross-section perpendicular to the yarn direction.
Fig 8: Example of a porosity and fibre gradient in a type B composite,
Fig 9: Glass fibres - PLA foam showing as well the set-up to place, orient and maintain fibres.
Fig 10: Continuous fibre reinforced foam microstructure. Fibre bundles are in between open and closed pores. Cross-section perpendicular to the yarn direction.
Fig 11: Continuous fibre reinforced foam microstructure. On the left, a cross-section parallel to the fibre direction and on the right a cross section through angle-oriented fibres.
Fig 12: Stress - strain curve of a given composite of type C.
Fig 13: Example of modulus and porosity ranges of a PLA foam reinforced with glass fibres.

### DETAILED DESCRIPTION

The present invention relates to a porous or cellular composite product also named composite foam which will be described in terms of unique microstructure, macrostructure, morphology and characteristics. The process to obtain such product is then presented with its own specifics.

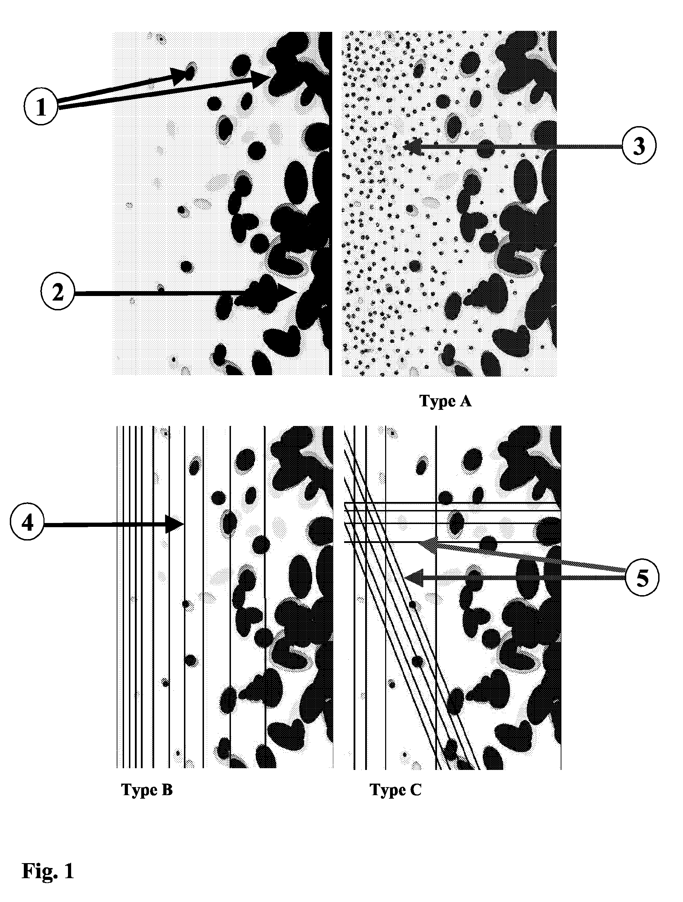

As depicted in Figure 1, the **composite** of this invention is a porous polymer matrix representing a continuous phase surrounding at least one additional material such as fillers or fibres. Cellular or porous structures exhibit open or closed pores, also named cells, (1) separated by walls (2). Reinforcement in the form of fillers, particles or short fibres (3), or continuous fibres in a 2D configuration (4) and in 3D architectures (5) are considered. They represent respectively types A, B and C of the novel cellular composites. Combinations of several reinforcement types are possible. The amount of fillers or fibres is relative to the amount of surrounding polymer and is expressed generally in volume fraction.

**Porosity** is defined in terms of relative volume of pores and of pore size distributions. When pores size and/or porosity volume change from one location to the other of the structure, a **gradient of porosity** is obtained. Different distribution functions can be used according to the variations of porosity volume desired in the different directions of the structure. Porosity can be closed and/or open when the pores are interconnected. The control of pore sizes, is important for applications where liquids or biological media are injected or where growth of living cells occurs into the porous composite.

**Distribution of fillers** can be uniform through the structure, but a variation in the volume fraction along at least one direction (Figure 1) generates a gradient of composition and performance which is desired in the context of this invention.
Fibres can be of different aspect ratios between their lengths and their diameters. With the increase of the aspect ratio various type of fibres are usually considered, from short fibres to long fibres and to continuous fibres. For the latter the length of the fibres is close to or larger than the length of the elements or the structure considered.
A precise placement of fibres is required to induce **gradients of fibre content or volume** and thus gradients of properties or gradients of functions when functional fibres are added. Several gradient types are obtained depending of the relative positions of the fibres.

The process described below will offer **different gradient types** in terms of linear or non linear variations of composition, volume fractions and properties in one direction at least, in terms of variations in several space directions and in terms of combination of gradients. For example a variation of porosity from high porosity in the center of a part to a low porosity in the outside skin of the part can be combined with low fibre content in the center and high fibre content in the outside region of the part.

The method of this invention to process the mentioned porous composites comprises two main steps: a) preparation and precise placement of the reinforcement or functional material, and b) in situ creation of porosity.

### a) Placement of reinforcement or functional material.

Described fillers and fibres are placed properly in a way that after the next processing step creating porosity, they will still be in the desired position to ensure gradient properties into the final cellular composite.

### Placement of fillers and short fibres

Fillers, short fibres, long fibres, extruded or preimpregnated compounds cut at desired lengths, are placed on a mould surface and distributed locally to generate variation of weight or volume fraction at different regions of the mould surface. Placement of fillers is made by hand or by using automated set-up (EP1184147A2). The preform is then moved to the next processing step to be transformed in a porous composite. Cellular composites of type A are based on this reinforcement placement and preform.

### Placement of continuous fibres

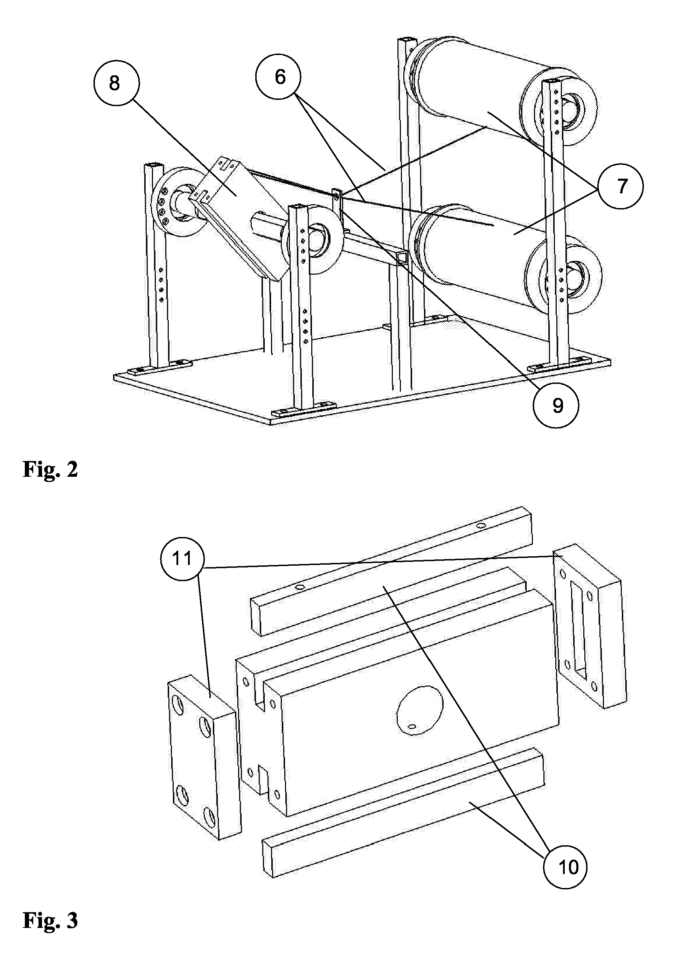

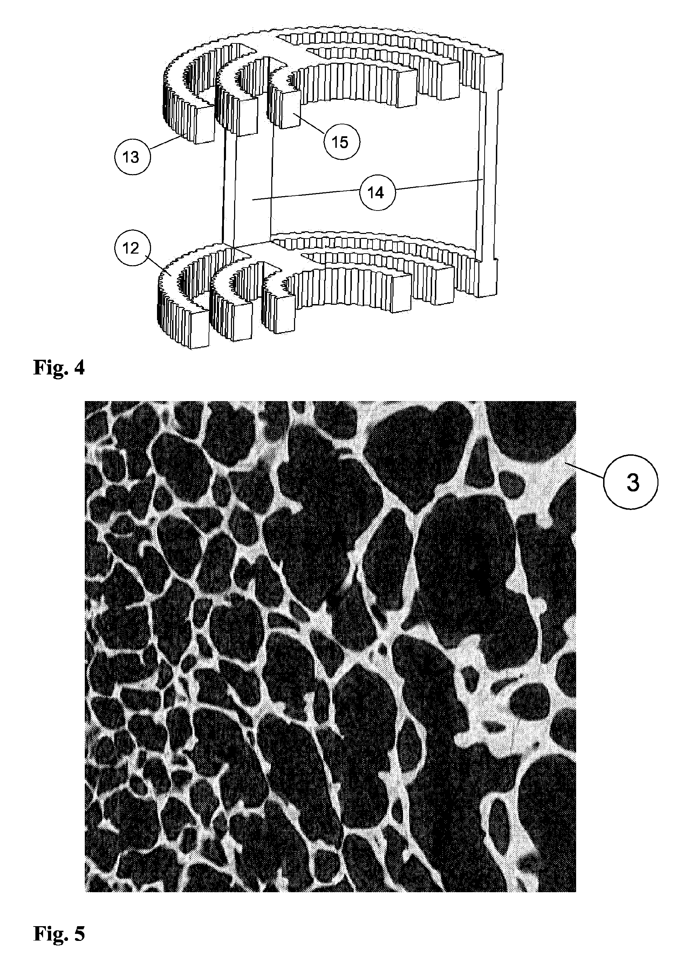

This processing step relates to a specific placement method to obtain cellular composites of type B and C.
For processing unidirectional type B composites a winding set-up (Figure 2) is developed for performing precise fibre or yarn placement to achieve smooth fibre distributions and thus gradients. Yarns or fibre bundles (6) are uncoiled from bobbins (7) and wound continuously onto a module (8) which serves as a mould for the next processing steps. This mould (8) can be of different geometries. Figure 3 and Figure 4 provide two examples. The driving force for the winding is a rotational motor allowing independent speeds for the mould and for each bobbin. Controlling this speed, the vertical fibre placement can be controlled. The fibre guiding devices (9) can be moved by linear motors and thus the horizontal or longitudinal fibre placement is controlled.

This equipment allows creating unidirectional composites with a precise cross-sectional yarns or fibres distribution. Furthermore the ratio of reinforcing and polymer filaments is controlled. Any 2D fibre volume gradient of one or more fibre types is achievable. Either reinforcement fibres such as dry fibres, preimpregnated fibres, commingled fibres coated fibres; or functional fibres such as foamable fibres, elastomeric fibres, fibres with any cross-sections, nano fillers based polymer fibres, optical fibres, metalic fibres or hybrid yarns of these fibres can be used. When the placed fibres are water sensitive, an appropriate drying step can be added. When filaments or fibre bundles are used, it is possible to build up commingled yarns directly on the mould.

To avoid any modification of the fibres position before the next consolidation or foaming step, the placed fibre bundles can be preconsolidated in a mould (Figure 3) without further movement of the placed fibres. The mould is placed for example between the plates of a heating press. The pressure is applied by two compression parts (10). Two stoppers (11) prevent the molten polymer from flowing away when the mould is heated for preconsolidation. The applied temperature, time and pressure provide a given level of preconsolidation, that is a given volume fraction of pores in the composite preform. To directly consolidate a composite with gradient properties, a conventional mould can also be used for the final consolidation of the preform. Any of the mentioned preimpregnated, preconsolidated or consolidated preforms can then be foamed as it is described below.

For processing composite foams comprising 3D fibre architecture (type C) the fibre holder depicted in Figure 4 is used. Mentioned reinforcement and functional fibres in a dry or preimpregnated state as well as tow and yarns can be fixed on the holder. Also a preform of the B-type composite as described above can be integrated here. Unidirectional, crossed, double crossed and numerous 3D fibre distributions and textile architecture can be realised or placed on this holder. Indeed, fibres can be aligned vertically or with different angles between the elements (12) and (13). These elements (12) and (13) can have other geometries, like being straight instead of curved like indicated in Figure 4. Furthermore fibres can be maintained horizontally between two or more elements (14). Fibres can thus have any orientation. By precisely varying the amount of fibres or yarns placed between the elements, gradients of fibre volumes are generated. For example a radial gradient is obtained by gradually placing more and more fibres when going from element (13) to element (15).

It is obvious that the holder can have more or less elements, can be of different sizes and that several of them can be combined. Furthermore, the holder can be used to maintain any types of fibre fabrics and textiles such as woven, knitted or braided preforms, dried or preimpregnated.

Together with this fibre holder, which guarantees that the reinforcement fibres stay stretched in place for the whole coming foaming step, the fibres may be dipped into a bath of liquid or molten matrix polymer for coating or pre-impregnation.
Afterwards the fibre holder and the fibres are put into a mould within the foaming chamber. When needed to complete desired polymer volume fraction, polymer powder or granulate is added to the mould to have enough matrix material.
In this way it is possible to process solvent-free thermoplastic foams, which are reinforced by oriented continuous fibres. Hereby the fibres orientation as well as the fibres distribution and fibres volume gradients can be chosen as one thinks best.

### b) Creation of porosity

### Control of consolidation

Porosity can be obtained by controlling the preconsolidation parameters of type B composite preform. By varying the heat transfer across the mould of Figure 3, controlling pressure level and time it is possible to create a porosity gradient in such a composite. Processing parameters are specific to each type of polymer material. Tests have been carried out indicating that porosity up to 20 % can be obtained in a controlled way with this technique.

### Gas foaming of composites

In the present invention a foaming process is proposed for the production of composite foams. The process avoids the use of any solvent or additional chemical foaming agent and allows the nucleation and growth of pores into a composite material while maintaining the position of the reinforcement. Subsequently, gradients of porosity and reinforcement content are obtained.

The prepared preforms based on the fillers or short fibres, as well as the preforms based on the continuous fibres are placed into a closed heated and pressurized vessel where the foaming process is induced. Supercritical CO₂ is used to foam the composite preforms placed into a hosting mold. Parameters are indicated here for a poly(L-lactic acid) based system, but can obviously be adjusted for any type of thermoplastic based system. CO₂ penetrates into the autoclave, and pressure is increased up to 50 to 300 bars, but more preferably between 100 and 250 bars. The gas saturation temperature Tₛₐₜ and pressure Pₛₐₜ, will control gas diffusion and concentration into the materials. Depressurization is obtained by gas release at a controlled flow rate, between 1 and 20 bar/s, and more preferably between 3 and 15 bar/s. Pores are nucleated. The foaming chamber is simultaneously cooled down due to depressurization and by additional water cooling. Cooling rates are preferably between 0.5 and 7.0 °C/s. Initial depressurisation rate dP/dt and maximum cooling rate dT/dt are significant parameters which influence pore expansion and stabilisation. The mentioned processing parameters are varied to control pore sizes and distributions in order to achieve different gradients of porosity. The presence of fibres influences the nucleation and growth of pores and thus requires specific foaming parameters. For example, higher saturation pressures and slower cooling rates are required to create interconnected porosity.

As they are mainly based on polymer, the moulded composite foam samples can be cut, machined or screwed for the specific requirements of given applications.

In conclusion, the invention relates to a process integrating the steps of placement and preparation of fillers or fibres preceding the step of foaming the composite preforms and thus obtaining porous composite structures with tailored gradients.

With the above mentioned methods and combinations of them porosity gradients with variations ranging between 0 to 90 % porosity in volume are achievable. At the same time, the fibre volume fraction, which is defined to be the fibre fraction within the solid material only can be tailored localy between 0 to 65 % to get different gradient types.
Thus depending on local porosity level and fibre volume fraction the mechanical properties such as the elastic modulus, can vary on a large range. Examples will provide values for specific material systems.

### MATERIAL SYSTEMS

Thermoplastic polymers in general can be used, such as polyethylene terephtalate (PET), polyethylene (PE), polyurethanes (PUR), etc. They can be reinforced with standard fillers or fibres using the mentioned processing steps.
Biocompatible and biodegradable polymers, fibres and particles already used in the biomedical field can be considered to prepare respectively biocompatible and biodegradable porous products to be used as scaffolds for example. Some examples of suitable polymers are α-polyhydroxy acids, such as poly(glycolic acid) (PGA), poly(lactic acid) (PLA, L or D,L enantiomers), poly(ε-caprolacton) (PCL), poly(trimethylene carbonate), poly(ethylene oxide) (PEO), poly(β-hydroxylbutyrate) (PHB), poly(β-hydroxyvalerate), poly(p-dioxanone) (PDS), poly(ortho esters), polypeptides, and copolymers of the above.
For the foaming process, polymers or compounds should have an intrinsic viscosity preferably higher than 0.8 dL/g, and more preferably higher than 1.0 dL/g. They must preferably be dried before processing, in order to prevent polymer hydrolysis.
Reinforcing elements can have the shape of particulate fillers, short, long or continuous fibres. They can be dry, preimpregnated with resins, coated or the result of a compounding process. Some suitable examples of ceramic particles are calcium phosphates, such as hydroxyapatite (HAp), β-tricalcium phosphate (β-TCP), calcium carbonate (CC), calcium dihydrogenphosphate (CDHP), calcium hydrogenphosphate (CHP), or mixtures of above, and bioactive glasses, such as Bioglass^{®}, phosphate based glasses.... Filler volume fraction is preferably comprised between 0 and 15 vol%. Composites of type A can also contain fillers, particles or fibres in the nanometric range such as nanofillers or nanotubes.
Long and continuous fibres can be made of traditional materials such as glass, carbon, of resorbable glass, such as Bioglass^{®} and phosphate based glasses. Polymer and composite fibres are also used. In opposite to filler material which increases the polymer's viscosity, the volume fraction of long/continuous fibres can go up to 65 %. The polymer's capacity for foaming is maintained even for such high fibre volume fractions because the polymer's viscosity is not changed. The polymer (eventually charged with a low fraction of fillers) will simply foam around the continuous fibres. Nevertheless the size and distribution of porosity is affected by fibre presence and distribution.

With the described method, combination of different fillers and fibre types is possible. For example, hybrid fibres systems, polymer fibres, biodegradable fibres, metallic fibres, optical fibres, functional fibres can be integrated into porous composite structures using the method of this invention. Degradable, foamable, coated or hollow fibres can bring additional porosity and additional ways to distribute pores into the final composite.

The composite foams obtained can be tailored in terms of porosity. Obviously they can thus be infiltrated and filled with any media, liquid or gel bringing additional function to the structure. For example bioactive or rheoactive fluids are of interest for tissue engineering applications and damping materials respectively.

### Example 1

### A composite product combining gradients of fillers and of porosity (type A)

The objective of this example is to illustrate how to obtain a composite porous structure of type A (Figure 1).
First 5 g of PLA + 5% β-TCP (PLA-5TCP), and 5 g of PLA + 10 % β-TCP (PLA-10TCP) are extruded under an inert atmosphere, using a micro-extruder (Micro5, DSM; the Netherlands). The following parameters are used: screw rotation speed 100 rpm, residence time 4 min and set temperature 200 °C. Extruded compounds are then dried and cut into 1 cm long rods. Into a 50 mm diameter cylinder mould, a paper cylinder of 35 mm diameter is placed inside. Inside this cylinder, the 5 g of PLA-5TCP are placed, and on the outside the 5 g of PLA-10TCP are added. The paper cylinder is then removed, leading to a gradient of β-TCP concentration in the composite.
In a second step, foaming is carried out. The mould prepared as previously described is put into the autoclave. After tightly closing, pressure is increased up to 200 bar, and temperature up to 195 °C. After 10 min saturation, pressure is released at a maximum rate of 4.5 bar/s, and cooling simultaneously occurs. Controlling the processing parameters, a porous composite structure with two gradients is achieved (Figure 5): first a higher β-TCP filler (3) concentration on the outside than in the core is obtained, due to the initial composite rods placement; second a porosity gradient is induced by a differential cooling on the outside and the core of the foam during processing.

### Example 2

### Composite product combining gradients of fibres and of porosity (Type B)

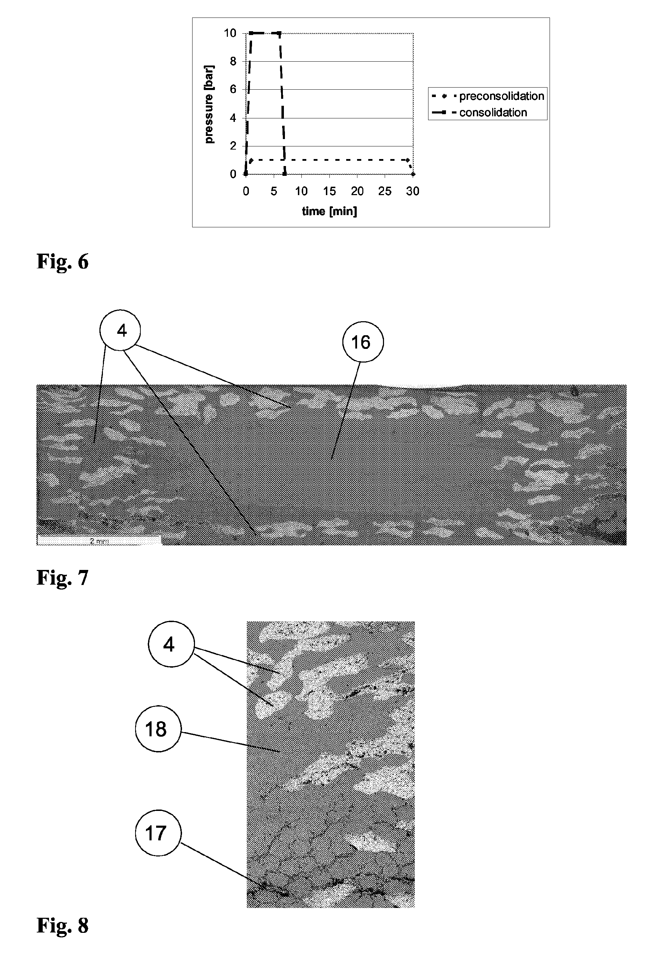

Processing steps and microstructure of type B composites with fibre volume gradients is described. The winding set-up is used to place polyamide (PA12) fibre bundles here with 32 monofilaments and carbon fibres (CF), in this case bundles of 250 monofilaments, around the mould (8) (Figures 2 and 3). The fibre volume fraction of carbon fibres at the external sides of the beam is 15%. Figure 6 depicts the processing parameters used for the preconsolidation and final consolidation when the thermoplastic material is processed at 200°C. For example, Figure 7 depicts the section of a Polyamide 12 (PA12) / carbon fibre (CF) composite having a fibre-free centre (16) surrounded by a fibre-rich region (4). The Figure 7 shows also that the relative position between fibre bundles could be maintained during the solidification steps. In horizontal and vertical directions, a bundle placement precision of less than 500 µm and 200 µm respectively was achieved with this material system.
Figure 8 illustrates a section of a PA 12 /CF composite with a porosity gradient induced by the control of the consolidation parameters, mainly the heat transfer on the mould. To achieve such a porosity gradient the mould is only heated on one side at 200°C. The other side is kept at room temperature which induces a temperature gradient throughout the preform. For 30 minutes a pressure of 1 bar is applied then. Playing with the parameters time, pressure and temperature, different porosity gradients can be processed. Porosity (17) is distributed into the polymer matrix (18) reinforced by a fibre gradient (4).

### Example 3

### Composite structure combining gradients of fibres and of porosity: type B and C.

The main challenge is here to combine fibre volume and porosity gradients, and more specifically, to preserve the initial fibre volume gradient when applying the gas foaming process to the system. The final fibre-reinforced foams have porosities ranging from 0 % or more up to 90 %.

The example is based on a bioresorbable polymer, Poly (L-lactic acid) (PLA) reinforced with glass fibres, but the method is not restricted to this material system.

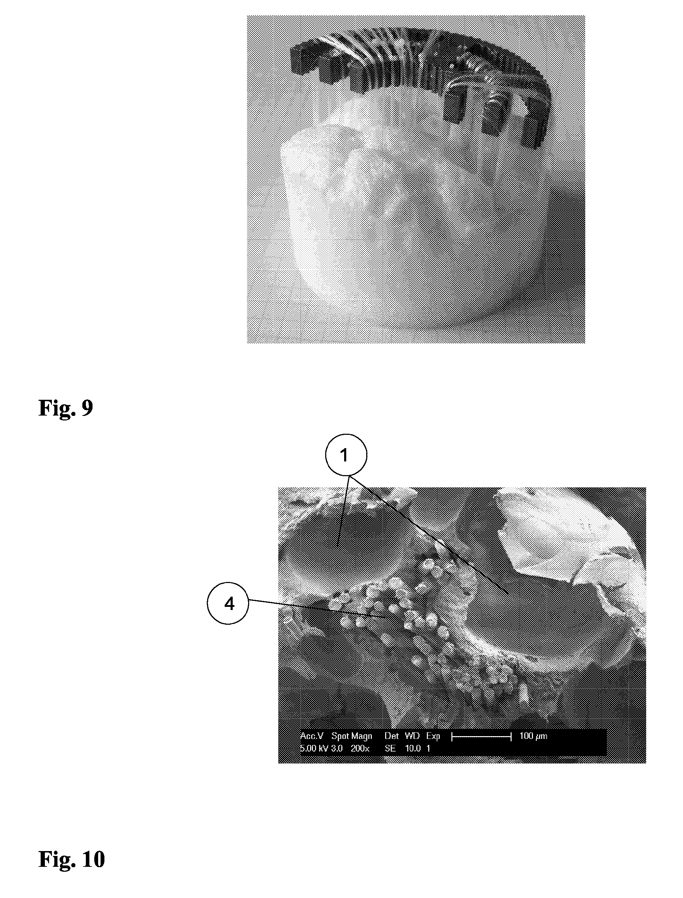

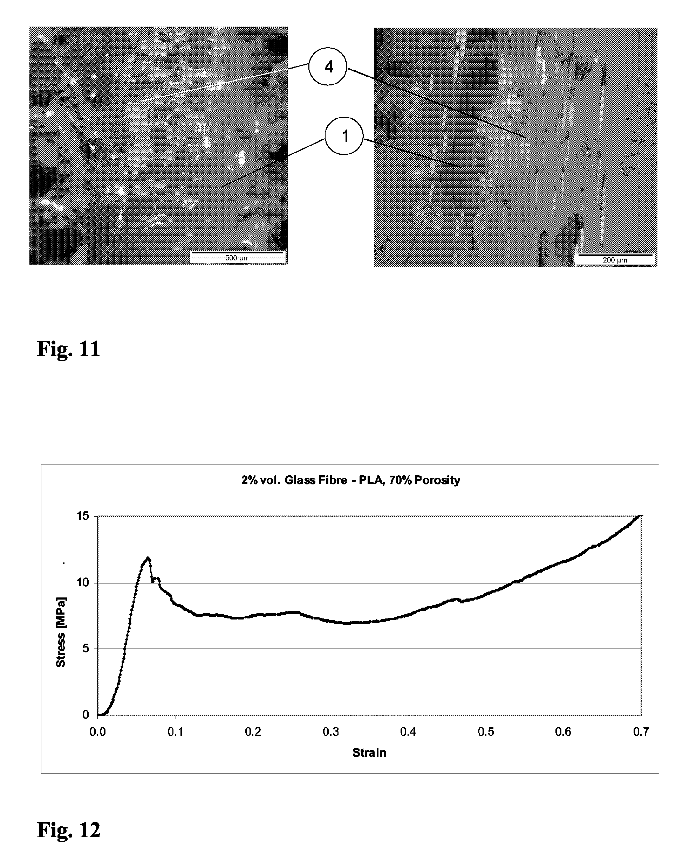

Continuous conventional E glass fibres are winded progressively around the holder (Figure 4) to form several layers with different fibres volumes and to keep fibres stable and oriented during foaming. The holder is then put into a cylindrical mould for the pressurised chamber, and the PLA pellets (15 g) are added to the fibres. Gas foaming is then carried out, using a saturation pressure of 200 bar. A glass fibre-reinforced PLA foam is thus obtained (Figure 9) with oriented fibres (4) situated in pore walls (Figure 10). Open and closed pores (1) and fibres are thus combined. Figure 11 illustrates another cross-section showing fibre (4) and porosity (1) distributions. Figure 12 is just one example of a stress-strain curve of PLA foam reinforced with 2 % vol. glass fibres and 70 % porosity. Compression was performed with a strain rate of 0,1 s⁻¹. An elastic modulus, E = 342 MPa, and an elastic collapse stress of 12 MPa were the measured characteristics of this specific foam.

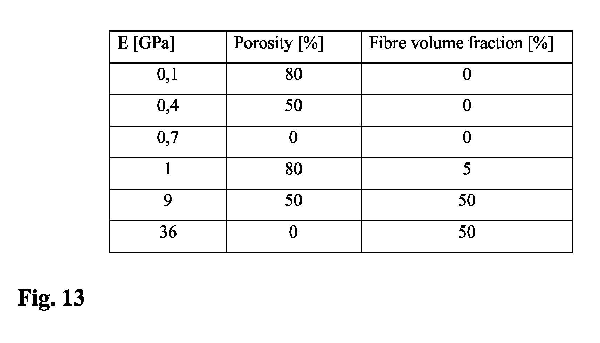

Depending on local porosity level and fibre volume fraction the mechanical properties such as the compression elastic modulus E can vary on a large range and in different gradient types. For the material system PLA and E glass fibres an overview of the achievable property range is given in Figure 13. The three first lines indicate how the modulus changes with a gradient of porosity going from 80 % down to 0%. The three last lines illustrate the increase of the modulus with the addition of a gradient going from 5 to 50 % of fibres volume content. This results show as well the properties when gradients of porosity and fibre volume fractions are combined.

In the examples discussed previously the processing of foams reinforced with fillers, short or long or continuous fibres and the resulting structures are demonstrated on an application in the medical field but the processing techniques allow also fabrication of such cellular gradient composites for other applications.

### REFERENCES

Atala, A. and D. J. Mooney (1997). Synthetic biodegradable polymer scaffolds. Boston.
Baksh, D. and J. E. Davies (2000). Design strategies for 3-Dimensional in vitro bone growth in tissue-engineering scaffolds. Bone Engineering. J. E. Davies.
Bledzki A.K., Z. W., Chate A. (2001). "Natural fibre-reinforced polyurethane microfoams." Composites Science and Technology 61: 2405-2411.
Bourban, P.-E., N. Bernet, et al. (2001). "Material phenomena controlling rapid processing of thermoplastic composites." Composite Part A : Applied science and manufacturing 32(8): 1045-1057.
Boyan, B. D., T. W. Hummert, et al. (1996). "Role of material surfaces in regulating bone and cartilage cell response." Biomaterials 17: 137-146.
Burg, K. J. L., S. Porter, et al. (2000). "Biomaterial developments for bone tissue engineering." Biomaterials 21(23): 2347-2359.
Chu, C. C. (2000). Biodegradable polymeric biomaterials. An updated review. The biomedical engineering handbook. J. D. Bronzino. Boca Raton, CRC Press. 1: 41.1-41.21.
Cima, L. G., J. P. Vacanti, et al. (1991). "Tissue engineering by cell transplantation using degradable polymer substrates." J Biomechanical Engineering 113: 143-149.
EP1184147A2 "Sheet impregnation unit and tow impregantion unit for the manufacture of fiber reinforced products."
Gibson, L. J. and M. F. Ashby (1988). Cellular solids- Structure and properties. Oxford, Pergamon Press.
Hutmacher, D. W. (2000). "Scaffolds in tissue engineering bone and cartilage." Biomaterials 21: 2529-2543.
Klempner, D. and K. C. Frisch (1991). Handbook of polymeric foams and foam technology. Munich, Hanser Publishers.
Laurencin, C. T., M. A. Attawia, et al. (1996). "Tissue engineered bone-regeneration using degradable polymers : the formation of mineralized matrices." Bone 19(1, supplement): 93S-99S.
Mainard, D., F. Gouin, et al. (2001). Les substituts osseux en 2001. Paris, Ed Romillat.
Manson, J. A. (2000). Comprehensive Composite Materials, Elsevier, Amsterdam.
Mathieu, L., T. Mueller, et al. (2006). "Architecture and properties of anisotropic polymer composite scaffolds for bone tissue engineering." Biomaterials 27: 905-916.
Mathieu, L. M., M. O. Montjovent, et al. (2005). "Bioresorbable composites prepared by supercritical fluid foaming." Journal of Biomedical Materials Research Part A 75A(1): 89-97.
Mathieu, L. M., M. T, et al. (2005). "Architecture and properties of anisotropic polymer composite scaffold for bone tissue engineering." biomaterials.
Piechota, H. and A. Röhr (1975). Integralschaumstoffe. Wien, Carl Hanser.
Rose, F. R. A. and R. O. C. Oreffo (2002). "Bone tissue engineering: Hope versus hype." Biochemical and Biophysical Research Communications 292: 1-7.
Shikinami, Y. and H. Kawarada (1998). "Potential application of a triaxial three-dimensional fabric (3-DF) as an implant." Biomaterials 19: 617-635.
Sunderland, P., W. Yu, et al. (2001). "A thermoviscoelastic analysis of process-induced internal stresses in thermoplastic matrix composites." Polym. Compos. 22(5): 579-592.
USpatent0054372A1 "Biodegradable composites."
USpatent4655777 "Method of producing biodegradable prosthesis and products therefrom."
USpatent5108755 "Biodegradable composites for internal medical use."
Wintermantel, E., J. Mayer, et al. (1996). "Tissue engineering scaffolds using superstructures." Biomaterials 17(2): 83-91.
Wolfrath, J., V. Michaud, et al. (2005). "Graded glass mat reinforced polypropylene." Polymer Composites 26(3): 361 - 369.

## Claims

1. A polymer composite product obtained by a process comprising the following steps :
- preparation of a composite preform by precise placement of dry or preimpregnated fillers and/or fibres in a mould,
- incorporation of the said preform and of a polymeric material in a foaming mould,
- foaming of the prepared composite,
- said polymer composite showing a gradient of filler and/or fibre content and a gradient of porosity.

2. A polymer composite product according to claim 1 wherein said process is a solvent-free process.

3. A polymer composite product according to claim 1 or 2 wherein variations of porosity are in at least one direction with local values of said porosity being between 0 and 90 %.

4. A polymer composite product according to anyone of the previous claims containing fibres wherein variations of fibre content are in at least one direction with local values of said content being between 0 and 65 %.

5. A polymer composite product according to anyone of the previous claims containing fillers wherein variations of filler content are in at least one direction with local values of said content being between 0 and 65 %.

6. A polymer composite product according to anyone of the previous claims wherein porosity is tailored for being filled with a media.

7. A polymer composite product according to anyone of the previous claims wherein porosity and mechanical performance are tailored for bone tissue engineering applications.

8. A polymer composite product according to anyone of the previous claims made of bioresorbable polymer and fibres.

9. A process for manufacturing a polymer composite product as defined in anyone of the previous claims, said process comprising the following steps :
- preparation of a composite preform by precise placement of dry or preimpregnated fillers and/or fibres in a mould,
- incorporation of the said preform and of a polymeric material in a foaming mould,
- foaming of the prepared composite

10. A process according to the previous claim wherein said process is a solvent-free process.

11. A process according to claim 9 or 10 wherein temperature, saturation pressure, depressurization rates and cooling rates are controlled to obtain tailored porosity, said porosity varying in at least one direction with local values comprised between 0 and 90%.

12. A process according to anyone of previous claims 9 to 11 using fibres wherein the fibres are distributed in such a way that in a least one direction the fibre content varies with local values comprised between 0 and 65 %.

13. A process according to anyone of previous claims 9 to 12 using fillers wherein the fillers are distributed in such a way that in a least one direction the filler content varies with local values comprised between 0 and 65 %.
